Europäisches Patentamt

European Patent Office

Office européen des brevets

(11) Publication number: **0 221 663**
A1

# EUROPEAN PATENT APPLICATION

(21) Application number: 86307348.2

(22) Date of filing: 24.09.86

(51) Int. Cl.4 **C12Q 1/02** , G01N 27/30 , C12M 1/40

(30) Priority: 25.09.85 GB 8523630

(43) Date of publication of application: 13.05.87 Bulletin 87/20

(84) Designated Contracting States: AT BE CH DE FR GB IT LI LU NL SE

(71) Applicant: **PAUL DE LA PENA LIMITED** Racecourse Road **Pershore Worcestershire WR10 2DD(GB)**

(72) Inventor: **Turner, Anthony Peter Francis** **Cranfield Inst. of Technology Biotechnology Cent.** **Cranfield Bedford, MK43 OAL(GB)** Inventor: **Franklin, Ann** **Cranfield Inst. of Technology Biotechnology Cent.** **Cranfield Bedford, MK43 OAL(GB)** Inventor: **Hodges, Alan Paul** **Paul de la Pena Ltd Racecourse Road** **Pershore Worcestershire, WR10 2DD(GB)** Inventor: **Ramsay, Graham** **Cranfield Inst. of Technology Biotechnology Cent.** **Cranfield Bedford, MK43 OAL(GB)** Inventor: **Steel, Davina** **Cranfield Inst.of Technology Biotechnology Cent.** **Cranfield Bedford, MK43 0AL(GB)**

(74) Representative: **Armitage, Ian Michael et al** **MEWBURN ELLIS & CO. 2/3 Cursitor Street** **London EC4A 1BQ(GB)**

(54) Methods and equipment for the bioelectrochemical cell measurement of microbial activity.

(57) A method of monitoring microbial activity in an aqueous analyte sample, which comprises combining a known volume of the sample with lyophilized bioelectrochemical cell components in a cell and measuring an electrical signal from electrodes in the cell. The use of lyophilized BEC components eliminates dilution of the sample, stabilizes the BEC components (e.g. ferricyanide), and increases the speed of response since the reagents rehydrate almost immediately.

Fig.1

# METHODS AND EQUIPMENT FOR THE BIOELECTROCHEMICAL CELL MEASUREMENT OF MICROBIAL ACTIVITY

This invention relates to the measurement of microbial activity by means of a bioelectrochemical cell - (BEC).

The use of BEC for collecting data on microbial activity has been investigated by a number of workers in recent years: see for example AU 498905; WO 82/04264; Matsunaga et al, Appl. Environ. Microbial. 37, II7-I2I (I979) and Eur. J. Appl. Microbial. I0, I25-I32 (I980); Turner et al, Biotechnology and Bioengineering Symposium No.I2 (I982) 40I-4I2; Turner et al, Biochemical Society Transactions, II, 445-448 (I983). The accurate determination of microbial activity is of considerable interest in, for example the food and drink industry, water and environmental pollution control, clinical analysis, including antibiotic sensitivity under clinical applications, etc. The present invention is directed to improvements in BEC methods and equipment for monitoring microbial activity.

According to one aspect of the present invention there is provided a method of monitoring microbial activity, which comprises combining a known volume of aqueous analyte sample with lyophilized BEC components in a cell and measuring an electrical signal from electrodes in the cell. The aqueous analyte sample may be provided by the analyte sample alone, or by the analyte sample and an additional quantity of aqueous medium to make up the known volume. The lyophilized components may be already present in the cell prior to the addition of the aqueous sample, or could be added subsequently.

In one embodiment, the lyophilized components are deposited on a surface within the cell, to which the aqueous sample is added directly. In another embodiment the lyophilized components are contained in a separate compartment within the cell to which aqueous medium is admitted after producing a suitable concentration of anaylte in the cell. The cell may contain a filter for the microbes, and initially a known, relatively large, quantity of analyte sample is passed through the filter before rehydrating the lypophilized components with the smaller known quantity of aqueous medium in the presence of the filtered microbes for operating the BEC.

In another aspect, the invention provides a pack of lyophilized components for BEC monitoring of microbial activity. The pack may take the form of a capsule or other unit to be added to the cell, or it may take the form of a cell containing the lyophilized components.

The lyophilized components typically may include the following (volumes indicated prior to lyophilization):

(i) KCl ImM Iml (to stabilize Ag/AgCl reference electrode).

(ii) Substrate:
e.g. Glucose 25 mM I50$\mu$l (a cell substrate of wide application).

The BEC could be made specific for certain (types) genera of bacteria by incorporating a substrate utilizable by only limited organisms:

e.g. (I) Lactate for lactic acid bacteria;

(2) Methanol for methanol degraders;

(3) Tricarboxylic acid cycle components for real samples (e.g. cutting fluids) containing bacteria not acclimatised to glucose.

(4) A relevant substrate in effluent treatment aiding assessment of degraders.

(iii) Mediator:
e.g. Ferricyanide 250 mM 500 $\mu$l

Ferricyanide is a mediator with wide applications, owing to its solubility and stability. Phenazine ethosulphate has possible applications in a light-protected cell. Another mediator useful in some applications is ferrocene and ferrocene derivatives, e.g. ferrocene monocarboxylic acid and other water-soluble derivatives.

Mediators preferably:
have relatively low redox potentials;
accept electrons from microorganisms;
exhibit reversible electrochemistry;
are chemically stable;
are non-auto-oxidizable;
are light-stable;
are normally soluble in aqueous solutions (except when immobilised, as below).

Specific mediators may:
be able in oxidized form to pass through or interact with the cell membrane, and in reduced form to pass out of the cell;
be immobilizable on the electrode.

Potassium or sodium ferricyanide are generally preferred at present. A cocktail of mediators can be used to give an even and sensitive response to a wide range of organisms.

(iv) Buffer:

Phosphate buffer pH 7.0 0.5M

Volume required is adjusted according to volume of other BEC components.

e.g. mediator : 500μl

KCL : lml

substrate : l50μl

water to l0 mls : 8.35 mls

Volume of phosphate buffer required is 835 μl to give final concentration: 50mM

Na or K phosphate buffers could be used.

Other components may also be included which help to enhance the BEC response, such as the following.

(v) Respiratory chain inhibitors and uncouplers may improve the BEC response by diverting the movement of electrons along the respiratory chain. (HOQNO)-2-n-heptyl-4-hydroxyquinoline-N-oxide is an example of an inhibitor. 2,4-dinitrophenol is an uncoupler of electron transport and energy conservation.

(vi) The BEC response may be further improved by facilitating the release of electrons from the bacteria by disrupting their cell wall to a greater or lesser extent by use of detergents; e.g. Triton-X-l00 or sodium dodecyl sulphate. Selective permeabilization of cell walls can be used to discrimiate between cells or between antibiotics.

The use of lyophilized BEC components has been found to have various advantages.

(a) It eliminates dilution which normally occurs on the addition of sample to a solution containing the BEC components.

(b) It stabilizes the BEC components, e.g. potassium ferricyanide mediator, which is unstable in solution over a prolonged period (<2 weeks even under refrigeration).

(c) It increases the speed with which a response is obtained: the freeze dried reagents rehydrate almost immediately on the addition of the aqueous sample, whereas dissolving the original solid chemicals can take a quite unacceptably long time, with the consequent risk of its not being done properly or at all before measuring the signal.

The invention is further illustrated by the following Examples, given by way of illustration only.

Example I

Fig. I shows a bioelectrochemical cell arrangement. The cell is contained in a beaker l0 with a lid ll, and placed in a water bath l2 with a magnetic stirrer l3. If desired, for example in the case of obligate anaerobes, nitrogen can be bubbled into the cell from a nitrogen line l4. The electrode l6 comprises a platinum electrode and a Ag/AgCl reference electrode. The output from the electrodes is taken to suitable data processing equipment. As illustrated, this comprises: a low noise current-to-voltage converter l8, a low noise by-pass filter 20, a digital volt meter 22, a buffer 24 and a precision voltage reference 26.

The BEC components to be freeze dried were made up in a l0ml beaker as usual.

KCl lmM lml

Glucose 25 mM 150μl

Ferricyanide 500μl

Phosphate buffer 0.5M pH 7.0 835μl

Total volume about 2.5ml

Sets of three beakers were prepared at each of 250mM, 50mM and 25mM ferricyanide concentrations.

The beakers were covered with Parafilm (trade mark) to prevent the loss of sample through bubbling in the freeze drying process, which was carried out overnight. Small holes were pierced through the parafilm to permit water vapour to be sucked away.

An overnight culture of milk isolate Yersiniaenterocolitica (biovar I), at an optical density of 0.875, was used to investigate the rehydration of freeze dried components. Cells (0.5ml) were routinely added together with water to make up to l0ml. A comparison was made between the response obtained using the lyophilized components and that obtained using the normal BEC. The BEC was set up as shown in Fig. I. At the same time a range of mediator concentrations was used to see what effect if any, reducing the mediator concentration had on the BEC response. The results are summarised in Table I.

## Table 1

## Effect of lyophilization on BEC response of milk isolate

## - Yersinia enterocolitica

| Lyophilized BEC components | Routine BEC set-up | Mediator concen- tration in BEC (Ferricyanide) | Initial slope µA/min |
|---|---|---|---|
| + | | 3.75 mM | 1.4 |
| + | | 0.75 mM | 1.76 |
| + | | 0.75 mM | 1.02 |
| | + | 0.75 mM | 1.36 |
| | + | 0.375 mM | 0.64 |
| + | | 0.375 mM | 0.80 |
| + | | 0.375 mM | 0.50 |

Several observations were made during these experiments. Firstly the lyophilized components rehydrated immediately without mixing on addition of the aqueous sample. Secondly the BEC response could be monitored within a very short time without the usual sparging of the solution with nitrogen for at least 5 minutes prior to measurement.

The standard mediator concentration in the BEC for this experiment is 3.75 mM although a concentration of l2.5 mM is used routinely for other BEC responses. In a rehydrated lyophilized sample with the bacteria added at the time of rehydration a measurable initial slope value of l.4µA/min was obtained with 2-3 minutes. The mediator concentration was decreased to 0.75mM and again upon rehydration initial slope values of l.76 and l.02µA/min were obtained. These values were of the same order of magnitude as in the 3.75mM ferricyanide concentration. It was deduced therefore, that the mediator concentration could be dropped to 0.75mM in the BEC without affecting the response, whilst at the same time diminishing background interference due to mediator oxidation. A standard BEC run using aqueous solutions was set up with 0.75mM ferricyanide and an initial slope reading of l.36µA/min was obtained in about l5 minutes. This showed that in this particular set of experiments, the use of dried BEC constituents was not reducing the BEC response obtained. The mediator concentration was further reduced to 0.375mM and the BEC responses obtained using rehydrated components were 0.8 and 0.5µA/min. Again a standard control BEC set up with 0.375mM ferricyanide gave a slope value of 0.64µA/min.

4

Whilst all three slope values were within the same range, they had dropped by at least 50%, in comparison with values obtained at 0.75mM ferricyanide concentration.

Example 2

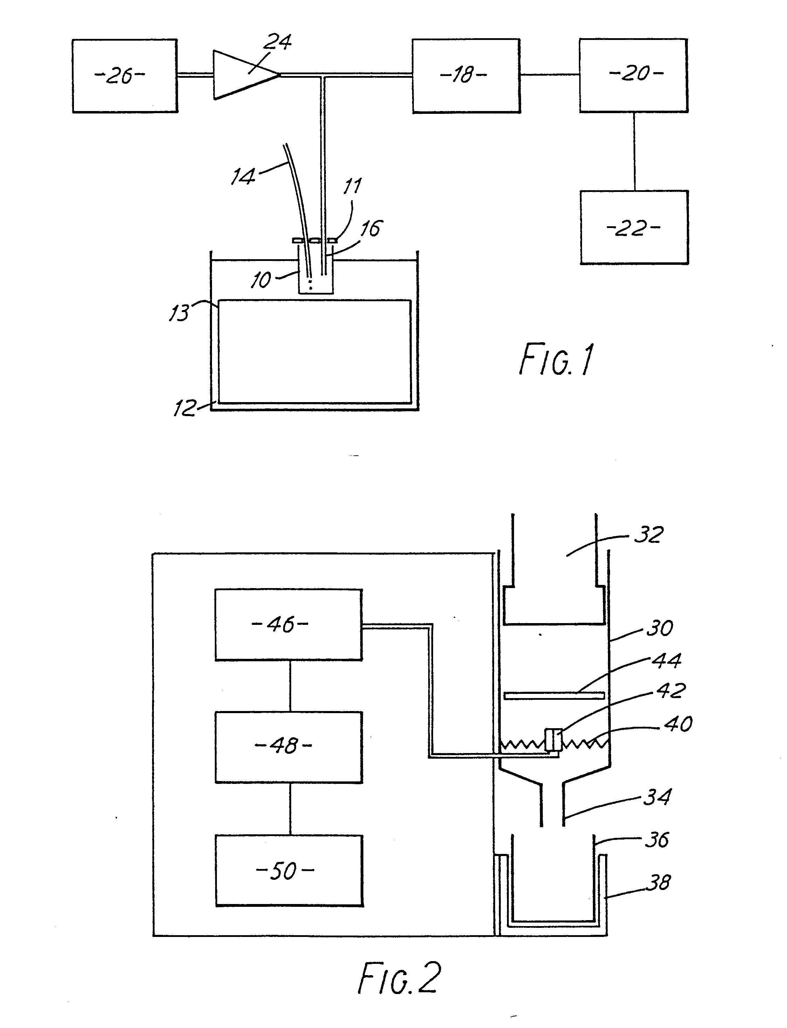

Fig. 2 shows another embodiment of BEC which can be used in accordance with the present invention. The cell is contained in a cylindrical housing 30, having a plunger 32 for drawing sample aqueous analyte through an inlet 34 from a beaker 36 optionally heated by a jacket 38. Within the cell is a porous support 40 on which has been previously deposited the lyophilized BEC components and surrounding the electrodes 42. Above the support and electrodes is a 0.22$\mu$m bacterial filter 44. The electrodes are connected to appropriate data processing equipment, such as an interface 46, microprocessor 48 and digital display 50.

In operation, the plunger 32 is withdrawn to introduce a known volume of aqueous analyte into the cell. The aqueous medium rehydrates the lyophilized components, and the cell produces an output from the electrodes indicating the level of microbial activity in the cell. When necessary, the bacterial filter 44 can confine the bacteria to the volume of the cell below the filter thus producing a concentrating effect thereby increasing the sensitivity of the measurement. In this case, the lyophilized components are added to the final volume of analyte sample in the cell. Of course, the reading from the electrodes has to be subjected to a conversion factor to obtain the level of bacterial activity in the original, more dilute, analyte sample, but this conversion can be effected by the microprocessor.

Example 3

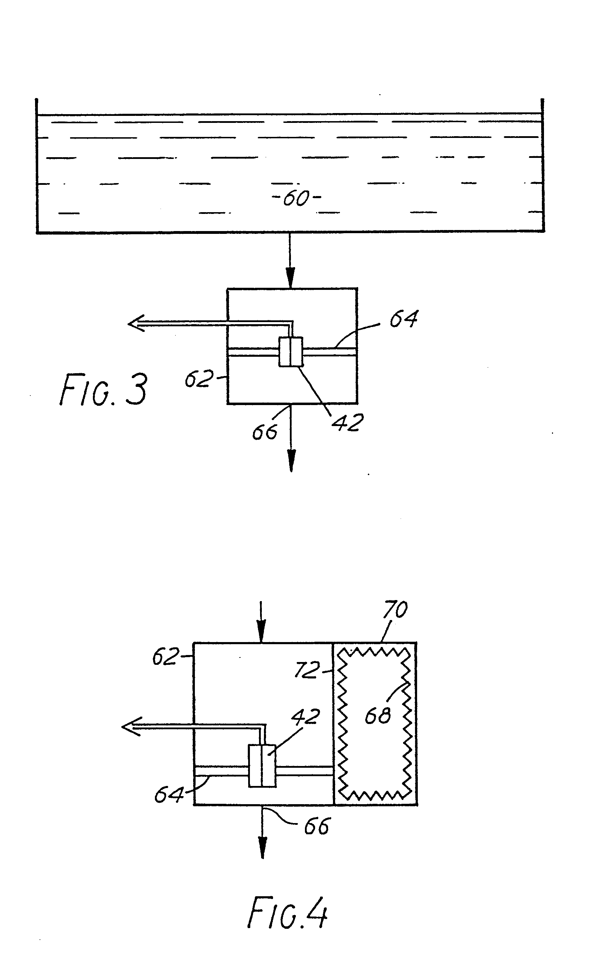

In the embodiment shown in Fig. 3, a large, but known, volume of aqueous analyte 60 is passed through the BEC 62, in which is a bacterial filter 64, and the electrodes 42 leading to the data handling equipment (not shown). When the desired volume of liquid has passed through, the outlet 66 to the cell is shut off with cell full of aqueous medium. Then a capsule or other pack containing the lyophilized BEC components is introduced into the cell, and the electrode measurements taken in the normal way.

This type of BEC procedure is particularly suitable for detecting low levels of contamination in large volumes of liquid, for example bacterial contamination of water.

Example 4

The embodiment shown in Fig. 4 comprises a BEC containing a bacterial filter for concentrating bacteria from aqueous analyte flowing through the cell in the same manner as in Fig. 3. However, the lyophilized components are contained on a support 68 in a compartment 70 adjacent to the cell and separated from the cell by a rupturable membrane 72. After concentrating the bacteria on the filter, the membrane is ruptured to introduce the lyophilized components into the cells, and readings can be taken as before.

Example 5

Into 10ml vials, 1ml of 1MKCl solution, 150$\mu$l of a 25mM glucose solution, 0.86ml of 0.5M phosphate buffer pH 7.0 and 0.5ml of a 20mM solution of ferrocene monocarboxylic acid (FcMCA) in 95% ethanol, were placed. The vials were covered with parafilm with small holes pierced in it and freeze dried overnight.

The vials were reconstituted with:
a) 9ml distilled water + 1ml 9% saline (control)
b) 9ml distilled water + 1ml of a mixed culture (resuspended in 9% saline)
c) 9ml distilled water + 1ml of Pseudomonas aeruginosa(in 9% saline)

Results -

| Sample | BEC Rate (μA/min) |
|---|---|
| a) Control | jump in current but no rate |
| b) Mixed culture | 1.54 |
| c) P. aeruginosa | 0.17 |

These results show that FcMCA was acting as a mediator after having undergone freeze drying.

Example 6

The freeze dried components were prepared as described above, with 500μl of 250mM potassium ferricyanide made up in distilled water replacing the FcMCA.

These freeze dried components were used to prepare a calibration curve of current at 59 seconds (μA) against E. coli concentration.

A simple hand-held device to provide a constant potential difference was used, and the current value obtained 59 seconds after rehydration of the BEC components with the E. coli suspension was noted. This experiment was carried out using a cylindrical platinum working electrode.

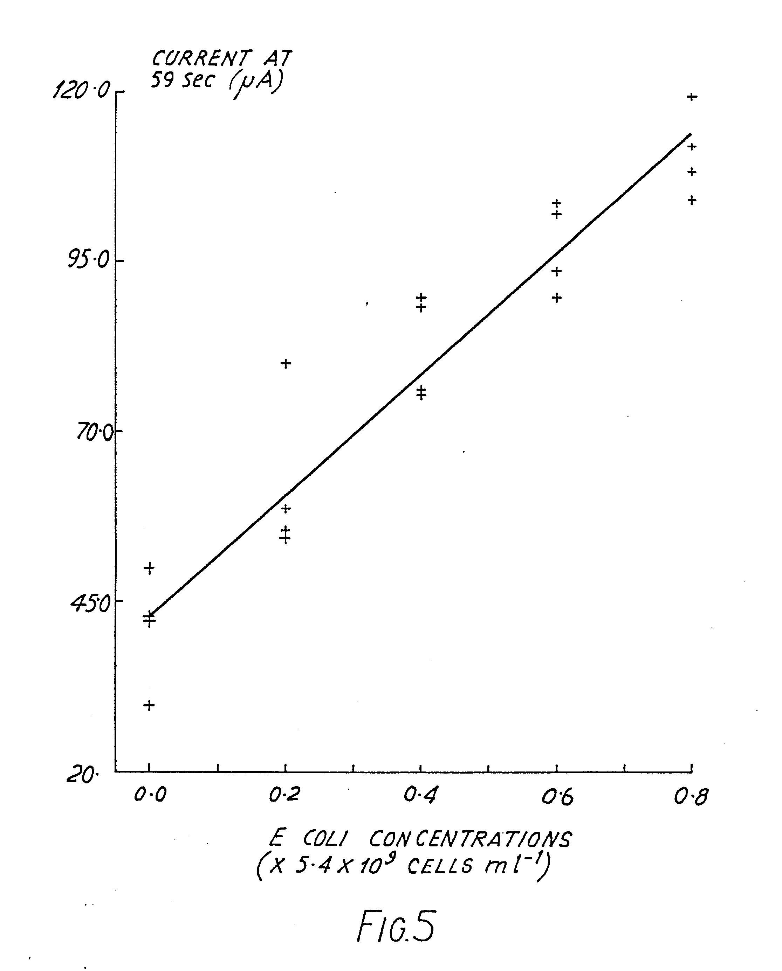

The graph of Fig. 5 showing current vs. concentration of E. coli, indicates the linear calibration observed. The linear regression plot correlation coefficient is 0.9529.

This experiment shows that ferricyanide functions as a mediator after having undergone freeze drying, and also emphasises the speed of response possible when freeze dried reagents are used.

**Claims**

I. A method of monitoring microbial activity in an aqueous analyte sample, which comprises combining a known volume of the sample with lyophilized bioelectrochemical cell components in a cell and measuring an electrical signal from electrodes in the cell.

2. A method according to claim I wherein the lyophilized components are pre-deposited on a surface within the cell, to which the sample is added.

3. A method according to claim 2 wherein the aqueous sample is added directly to the lyopholized components within the cell.

4. A method according to claim 2 wherein the lyophilized components are contained in a separate compartment within the cell, and aqueous sample medium is admitted to the compartment after concentrating the analyte in the cell.

5. A method according to claim 4 wherein a volume of sample larger than the cell is passed through a microbe filter in the cell to concentrate the microbes prior to contacting the microbes with the lyophilized components in a known volume of aqueous medium.

6. A method according to claim I wherein a volume of sample larger than the cell is passed through a microbe filter in the cell to concentrate the microbes, and the lyophilized components are added to the cell containing the concentrated microbes in a known volume of aqueous medium.

7. A pack of lyophilized bioelectrochemical cell components which, on contact with an aqueous analyte sample in a cell, produce a bioelectrochemical cell electrical output resulting from microbial activity in the sample.

8. A bioelectrochemical cell containing lyophilized components which, on contact with an aqueous analyte sample, produce an electrical output from the cell resulting from microbial activity in the sample.

9. A method, pack or cell according to any one of the preceding claims wherein the lyophilized components include a mediator selected from ferricyanide, ferrocene, ferrocene monocarboxylic acid, other ferrocene derivatives, and mixture thereof.

FIG.1

FIG.2

FIG. 3

FIG.4

FIG.5

0 221 663

**European Patent Office**

**EUROPEAN SEARCH REPORT**

Application number

EP  86 30 7348

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,A | AU-A- 498 905 (UNIVERSITY OF VIRGINIA AND NATIONAL AERONAUTICS AND SPACE ADMINISTRATION) <br> * complete * | 7 | C 12 Q 1/02 <br> G 01 N 27/30 <br> C 12 M 1/40 |
| D,A | WO-A-8 204 264 (ALFALAVAL) <br> * complete * | 1 | |
| A | US-A-4 168 204 (W. WILLIAMS) | | |
| P,A | EP-A-0 161 481 (TERUMO K.K.) | | |
| A | BIOTECHNOLOGY ADVANCES, vol. 3, no. 2, 1985, pages 209-218, Oxford, GB; A. GRAHAM et al.: "Biosensors: Recent trends" | | **TECHNICAL FIELDS SEARCHED (Int. Cl 4)** <br><br> C 12 Q 1/00 <br> G 01 N 27/00 <br> C 12 M 1/00 |
| A | EP-A-0 078 636 (GENETICS INTERNATIONAL) <br> * examples 1-8 * | 1,9 | |

The present search report has been drawn up for all claims

| Place of search | Date of completion of the search | Examiner |
|---|---|---|
| BERLIN | 16-12-1986 | GREEN C.H. |